# FASCICULE DE BREVET EUROPEEN

(11) **EP 1 589 906 B1**
(45) Date de publication et mention de la délivrance du brevet: **22.04.2009**
(21) Numéro de dépôt: 04708377.9
(22) Date de dépôt: 05.02.2004
(51) Int. Cl.: A61F 2/30, A61F 2/44, A61F 2/46

(54) **IMPLANT DE REMPLACEMENT VERTEBRAL**
WIRBELKÖRPERERSATZVORRICHTUNG
VERTEBRAL REPLACEMENT DEVICE

(30) Priorité: 05.02.2003 FR 0301339
(43) Date de publication de la demande: 02.11.2005
(73) Titulaire: SCIENT'X, 78284 Guyancourt (FR)
(72) Inventeur: ASSAKER, Richard, B-7540 Kain (BE)
(74) Mandataire: Thibault, Jean-Marc
(86) Numéro de dépôt international: PCT/FR2004/000262
(87) Numéro de publication internationale: WO 2004/071355

(56) Documents cités:
- WO-A-99/38462
- WO-A-99/63913
- DE-A- 19 856 013
- US-A1- 2002 082 696
- US-B1- 6 176 881

## Description

La présente invention concerne un implant médical destiné à remplacer, au moins partiellement, une vertèbre en vue de maintenir l'écartement normal entre les vertèbres adjacentes.

L'objet de l'invention trouve une application pour le remplacement vertébral au niveau cervical, thoracique ou lombaire.

Dans l'état de la technique, il est connu diverses variantes de réalisation d'un implant de remplacement appelé également implant de corporectomie. Par exemple, la demande de brevet FR 2 730 158 décrit un implant de remplacement comprenant deux éléments creux emboîtables l'un dans l'autre et présentant au niveau de leur face mutuellement en regard, des formes complémentaires coopérant entre elles pour permettre le déplacement en distraction des éléments tout en évitant leur déplacement en compression.

Un tel implant de remplacement permet de pouvoir régler in situ la longueur de .. l'implant en assurant l'écartement relatif entre les deux éléments par coulissement jusqu'à leur position définitive de blocage. Un tel implant possède ainsi un blocage irréversible interdisant une possibilité de rétraction, ce qui constitue un inconvénient majeur dans certaines conditions de mise en place.

Le document WO 00/23 013 décrit un implant de remplacement comportant deux éléments longitudinaux creux mâle et femelle montés coulissants l'un par rapport à l'autre. Cet implant de remplacement comporte une bague de couplage destinée à occuper une première position de non-coopération avec un moyen complémentaire de couplage pour autoriser un libre coulissement des éléments entre eux, et une deuxième position de coopération avec le moyen complémentaire de couplage pour assurer un blocage des éléments entre eux. L'inconvénient principal d'un tel implant de remplacement a trait à la difficulté de commander en rotation la bague de couplage pour passer d'une position à l'autre.

L'objet de l'invention vise à remédier aux inconvénients de l'art antérieur en proposant un implant de remplacement vertébral conçu pour permettre d'être réglé en longueur in situ tout en permettant de passer d'une manière simple, rapide et réversible d'une position de réglage en longueur à une position de blocage.

Le document US 2002/082696 décrit un implant de remplacement vertébral comportant deux éléments creux mâle et femelle montés coulissants l'un par rapport à l'autre. Cet implant comporte des moyens assurant le blocage et le libre coulissement des éléments entre eux. Ces moyens de blocage comportent une série d'indentations aménagée sur une partie longitudinale de l'élément mâle tandis que l'élément femelle comporte un trou taraudé de réception pour une vis de blocage adaptée pour coopérer avec les indentations.

Pour atteindre un tel objectif, l'implant de remplacement vertébral est conforme à la revendication 1.

Avantageusement, l'axe s'étend diamétralement à l'intérieur de l'élément mâle et comporte :
- une première extrémité pourvue du moyen de réception de l'instrument de commande, cette première extrémité étant guidée en rotation et bloquée en translation dans une paroi de l'élément mâle,
- une deuxième extrémité munie de la partie filetée coopérant avec le taraudage du patin d'ancrage qui est guidé en translation et bloqué en rotation dans la paroi opposée de l'élément mâle.

Avantageusement, le moyen de réception de l'instrument de commande débouche dans une lumière aménagée dans une paroi de l'élément femelle pour être accessible de l'extérieur de l'implant, la paroi de l'élément femelle étant munie intérieurement dans sa partie opposée de celle pourvue de la lumière, de la surface d'ancrage.

De préférence, chaque élément mâle et femelle est pourvu d'une paroi d'extrémité destinée à coopérer avec un plateau vertébral.

Un autre objet de l'invention est de proposer un implant de remplacement permettant d'optimiser le contact entre au moins une des parois d'extrémité de l'implant et le plateau vertébral. Pour atteindre un tel objectif, au moins une paroi d'extrémité est équipée d'un système d'inclinaison dans le plan sagittal.

Avantageusement, le système d'inclinaison est du type vis-écrou et comporte une tige filetée guidée en rotation et bloquée en translation pour coopérer avec une bague taraudée bloquée en rotation et reliée à la paroi d'extrémité guidée en inclinaison.

Avantageusement, la tige filetée s'étend diamétralement à l'intérieur d'un élément et comporte un moyen de réception d'un instrument de commande accessible de l'extérieur de l'élément.

Avantageusement, le dispositif anti-retour est débrayable autorisant le mouvement selon un sens de rapprochement des éléments mâle et femelle.

Diverses autres caractéristiques ressortent de la description faite ci-dessous en référence aux dessins annexés qui montrent, à titre d'exemples non limitatifs, des formes de réalisation de l'objet de l'invention.

La **Fig. 1** est une vue en perspective montrant un exemple de réalisation d'un implant conforme à l'invention.

La **Fig. 2** est une vue en coupe élévation montrant l'implant vertébral dans une position de blocage.

La **Fig. 3** est une vue partielle en coupe élévation montrant l'implant en position de libre coulissement des éléments entre eux.

La **Fig. 4** est une vue en perspective et en partie arrachée montrant des détails caractéristiques de l'objet de l'invention.

La **Fig. 5** est une vue en coupe élévation d'une caractéristique préférée de réalisation équipant un implant vertébral conforme à l'invention.

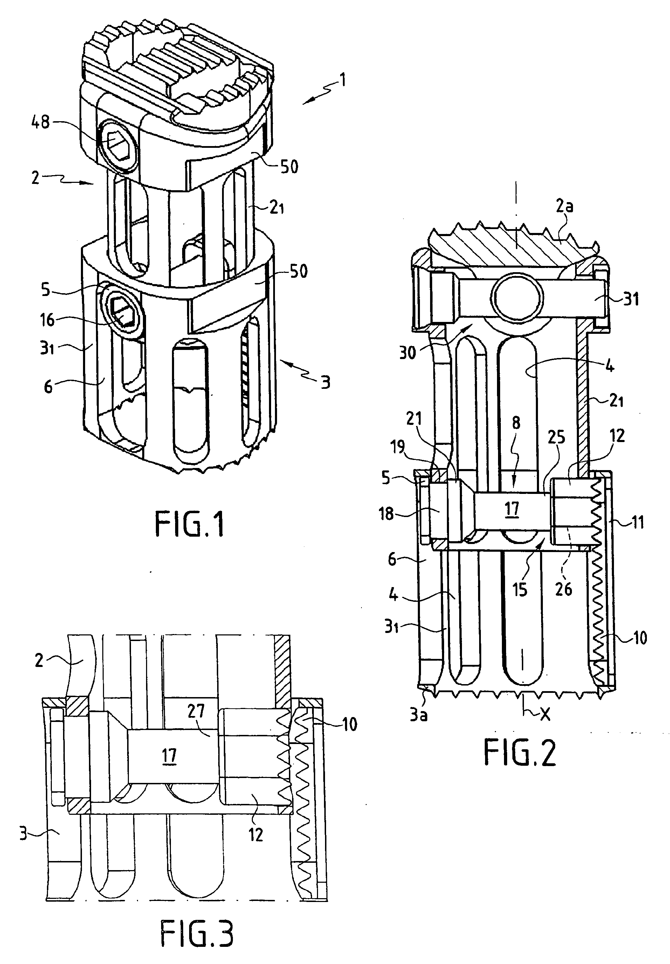

Tel que cela ressort plus précisément des **Fig. 1 à 4****,** l'objet de l'invention concerne un implant de remplacement vertébral **1** comportant un premier élément longitudinal creux dit mâle **2** et un deuxième élément longitudinal creux dit femelle **3.** Chaque élément **2, 3** comporte respectivement un corps **2₁, 3₁** de forme générale tubulaire s'étendant selon un axe longitudinal **X.** Le corps mâle **2₁** est adapté pour être engagé partiellement à l'intérieur du corps femelle **3₁** de manière à s'étendre coaxialement l'un à l'autre. Le corps mâle **2₁** possède une enveloppe externe de section et de forme sensiblement identiques au jeu près, à l'alésage interne délimité par le corps femelle **3₁.** De préférence, les corps mâle **2₁** et femelle **3₁** comportent des évidements **4** pour le passage d'un volume d'ancrage osseux.

Selon une caractéristique de l'invention, les éléments mâle **2** et femelle **3** sont montés coulissants l'un par rapport à l'autre selon leur axe commun longitudinal **X.** Dans l'exemple illustré, le montage coulissant entre les éléments mâle **2** et femelle **3** est réalisé par l'intermédiaire d'une butée de guidage **5** équipant l'élément mâle **2** et engagée dans une lumière ouverte **6** aménagée sur l'élément femelle **3** selon une direction longitudinale parallèle à l'axe longitudinal **X.**

Selon une caractéristique de l'invention, l'implant de remplacement **1** comporte des moyens **8** assurant d'une part, le coulissement libre des éléments **2, 3** entre eux et d'autre part, leur blocage relatif. Ces moyens de blocage **8** comportent sur l'un des éléments à savoir l'élément femelle **3** dans l'exemple illustré, une surface d'ancrage **10** aménagée sur une partie longitudinale **11** de la paroi. Cette surface d'ancrage **10** s'étend à l'intérieur de la paroi de l'élément femelle **3** en vis-à-vis d'un patin d'ancrage **12** monté sur l'autre élément à savoir l'élément mâle **2** dans l'exemple illustré. Le patin d'ancrage **12** est guidé en translation pour occuper une première position dans laquelle les éléments mâle **2** et femelle **3** peuvent coulisser librement entre eux **(****Fig. 3****)** et une deuxième position dans laquelle le patin d'ancrage **12** coopère avec la surface d'ancrage **10** pour bloquer en position les éléments **2, 3** entre eux **(****Fig. 2****).** A cet effet, le patin d'ancrage **12** comporte sur sa face transversale s'étendant perpendiculairement à la direction de translation du patin, une forme d'ancrage complémentaire ou conjuguée à la surface d'ancrage **10** de manière qu'en coopération mutuelle, les éléments **2, 3** se trouvent verrouillés en position. Par exemple, le patin d'ancrage **12** et la surface d'ancrage **10** présentent des dents s'étendant transversalement par rapport à la direction de coulissement du patin d'ancrage **12.**

Selon une autre caractéristique de l'invention, les moyens de blocage **8** comportent également un système **15** de transformation d'un mouvement de rotation en un mouvement de translation qui est transmis au patin d'ancrage **12** de manière qu'il occupe sa position dégagée ou sa position de coopération avec la surface d'ancrage **10.** Le système de transformation **15** est équipé d'un moyen **16** de réception d'un instrument non représenté, adapté pour commander en rotation le système **15** selon les deux sens opposés. Ainsi, en fonction du sens de commande en rotation imposé par l'instrument de commande, le patin d'ancrage **12** est apte à occuper une de ses deux positions caractéristiques.

Dans un exemple préféré de réalisation illustré par les dessins, le système de transformation **15** est du type vis-écrou. Selon cet exemple préféré de réalisation, le système de transformation **15** comporte un axe **17** guidé en rotation et bloqué en translation et monté pour s'étendre diamétralement à l'intérieur de l'élément mâle **2,** dans sa partie restant toujours engagée dans l'élément femelle. L'axe **17** comporte une première extrémité **18** de section droite circulaire montée avec jeu, dans un alésage **19** réalisé dans la paroi de l'élément mâle **2.** La première extrémité **18** est bordée d'un côté par un épaulement **21** et de l'autre côté par la butée **5.** L'axe **17** est ainsi bloqué en translation par la butée **5** et l'épaulement **21,** tout en étant guidé en rotation.

Avantageusement, la première extrémité **18** de l'axe **17** est pourvue du moyen de réception **16** de l'instrument de commande en rotation de l'axe. Dans l'exemple illustré, le moyen de réception **16** est réalisé par un trou de section prismatique aménagé sur la face transversale délimitant la première extrémité de l'axe **17.** Le trou 16 s'ouvre dans la lumière débouchante **6** s'ouvrant sur la face externe de l'élément femelle **3** de manière que le trou **16** puisse être accessible de l'extérieur de l'implant à l'aide d'un instrument de commande tel qu'un tournevis par exemple.

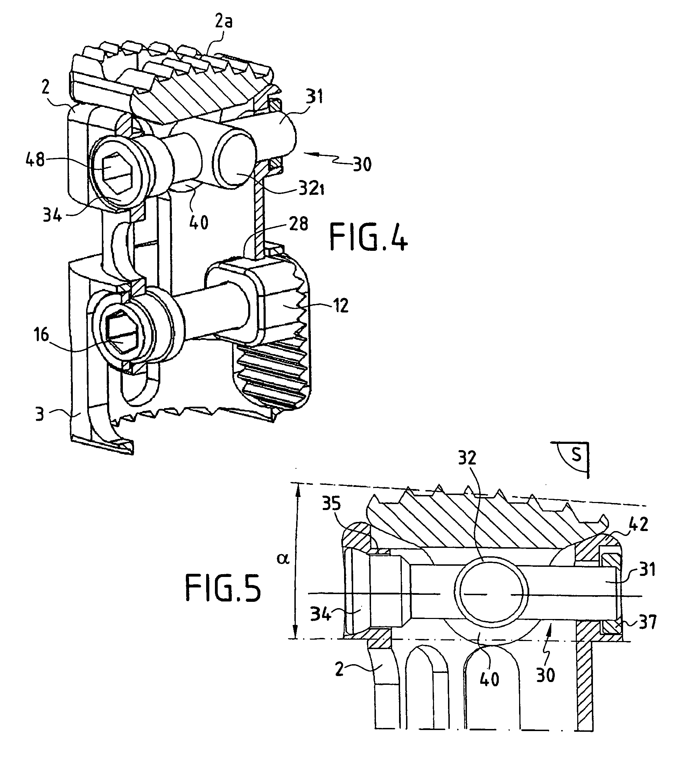

L'axe **17** est pourvu d'une partie filetée **25** coopérant avec un taraudage **26** présenté par le patin d'ancrage **12** qui est guidé en translation et bloqué en rotation. Dans l'exemple illustré, la deuxième extrémité **27** de l'axe **17** opposée à la première extrémité **18,** est pourvue de la partie filetée **25.** Ainsi, tel que cela ressort plus précisément de la **Fig. 4****,** le patin d'ancrage **12** présente une forme extérieure prismatique montée à l'intérieur d'un alésage **28** de section analogue aménagé dans la paroi de l'élément mâle **2** assurant ainsi un guidage en translation et le blocage en rotation du patin d'ancrage **12.** Tel que cela ressort de la description qui précède, la rotation de l'axe **17** dans un sens permet le coulissement du patin d'ancrage **12** pour le rapprocher de la surface d'ancrage **10** tandis que la rotation de l'axe **17** dans un sens opposé conduit à écarter par translation le patin d'ancrage **12** relativement à la surface d'ancrage **10.**

Selon une autre caractéristique de l'invention, chaque élément mâle **2** et femelle **3** est pourvu d'une paroi d'extrémité **2ₐ, 3ₐ** destinée à coopérer avec un plateau vertébral. De préférence, chaque paroi d'extrémité **2ₐ, 3ₐ** est pourvue d'un crantage pour favoriser l'ancrage de l'implant sur les plateaux des vertèbres sus et sous jacentes.

Selon une variante préférée de réalisation, au moins une paroi d'extrémité, à savoir la paroi d'extrémité **2ₐ** portée par l'élément mâle **2** dans l'exemple illustré est équipé d'un système **30** assurant son inclinaison dans le plan sagittal anatomique représenté par la référence **S** sur les dessins. Selon une caractéristique préférée de réalisation, le système d'inclinaison **30** est du type vis-écrou. Ce système d'inclinaison **30** comporte une tige filetée **31** guidée en rotation et bloquée en translation pour coopérer avec une bague taraudée **32** bloquée en rotation et reliée à la paroi d'extrémité **2ₐ** dans l'exemple illustré. Plus précisément, la tige filetée **31** s'étend diamétralement à l'intérieur de l'élément mâle **2** selon une direction parallèle à la direction d'extension de l'axe **17.** La tige filetée **31** est pourvue à une extrémité d'une tête **34** traversant par un passage **35** la paroi de l'élément mâle **2.** L'autre extrémité de la tige filetée **31** est équipée d'une butée d'arrêt en translation **37.** La tige filetée **31** coopère avec la bague taraudée **32** qui est pourvue de part et d'autre de prolongements **32₁** engagés dans des trous complémentaires aménagés dans des pattes **40** s'étendant à l'équerre à partir de la paroi d'extrémité **2ₐ.**

Tel que cela ressort plus précisément des **Fig. 4 et 5****,** la paroi d'extrémité **2₈** est supportée par un plateau **42** s'étendant à partir du corps de l'élément mâle **2** et délimitant par son rebord supérieur un berceau de guidage en rotation pour la paroi d'extrémité **2ₐ.** Ainsi, la paroi d'extrémité **2₈** présente une face interne convexe apte à coopérer avec le plateau **42** de forme concave.

Tel que cela ressort clairement de la comparaison des **Fig. 2** et **4****,** la rotation de la tige filetée **31** conduit à une translation de la bague taraudée **32** qui impose une rotation de la paroi d'extrémité **2ₐ** guidé par le berceau de l'élément mâle **2.** La paroi d'extrémité **2ₐ** peut ainsi être incliné d'un angle α par exemple de l'ordre de 5°.

Selon une caractéristique de l'invention, la tige filetée **31** comporte un moyen de réception **48** d'un instrument de commande accessible de l'extérieur de l'élément mâle **2.** Dans l'exemple illustré, le moyen de réception **48** est réalisé par un trou de section prismatique aménagé dans la paroi transversale délimitant la tête **34** de la tige filetée **31.** Le trou **48** est donc accessible de l'extérieur de l'élément mâle **2** et se trouve de préférence aligné avec le moyen de réception **16** selon la direction longitudinale. Les moyens de réception **16** et **48** qui occupent une position superposée proche, sont de préférence de forme identique de manière à pouvoir être manoeuvrés par un même instrument de commande tel qu'un tournevis par exemple.

Selon une autre caractéristique de l'invention, l'implant intervertébral **1** est adapté pour assurer le déplacement en coulissement de manière simple et efficace des éléments mâle **2** et femelle **3.** A cet effet, chaque élément mâle **2** et femelle **3** comporte des zones **50** de préhension pour un appareil de distraction assurant le coulissement relatif entre les éléments mâle **2** et femelle **3.** Ces zones de préhension **50** sont réalisées sur chaque élément, par deux méplats s'étendant de façon diamétralement opposée sur les éléments mâle **2** et femelle **3.**

L'invention n'est pas limitée aux exemples décrits et représentés car diverses modifications peuvent y être apportées sans sortir de son cadre.

## Revendications

1. Implant de remplacement vertébral comportant :
• deux éléments longitudinaux creux dits mâle **(2)** et femelle **(3)** montés coulissant l'un par rapport à l'autre selon un axe commun longitudinal **(X),**
• et des moyens **(8)** assurant le blocage et le libre coulissement des éléments entre eux, les moyens de blocage **(8)** comportant :
- sur l'un des éléments, une surface d'ancrage **(10)** aménagée sur une partie longitudinale dudit élément,
- et sur l'autre des éléments :
un patin d'ancrage guidé **(12)** en translation pour occuper une première position dans laquelle les éléments **(2, 3)** coulissent librement entre eux et une deuxième position dans laquelle le patin d'ancrage **(12)** coopère avec la surface d'ancrage **(10)** pour bloquer les éléments **(2, 3)** entre eux,
un système **(15)** de transformation d'un mouvement de rotation en un mouvement de translation transmis au patin d'ancrage **(12),** le système de transformation de mouvement **(15)** étant pourvu d'un moyen de réception **(16)** d'un instrument de commande en rotation selon les deux sens opposés de sorte que le patin d'ancrage **(12)** occupe une de ses positions en fonction du sens de commande en rotation,
, **caractérisé en ce que** le système de transformation est du type vis-écrou (15) et comporte un axe **(17)** guidé en rotation et bloqué en translation, cet axe **(17)** étant pourvu d'une partie filetée **(25)** coopérant avec un taraudage **(26)** présenté par le patin d'ancrage **(12)** guidé en translation et bloqué en rotation.

2. implant de remplacement vertébral selon la revendication 1, **caractérisé en ce que** l'axe **(17)** s'étend diamétralement à l'intérieur de l'élément mâle **(2)** et comporte :
• une première extrémité **(18)** pourvue du moyen **(16)** de réception de l'instrument de commande, cette première extrémité étant guidée en rotation et bloquée en translation dans une paroi de l'élément mâle,
• une deuxième extrémité **(27)** munie de la partie filetée **(25)** coopérant avec le taraudage **(26)** du patin d'ancrage qui est guidé en translation et bloqué en rotation dans la paroi opposée de l'élément mâle.

3. Implant de remplacement vertébral selon la revendication 2, **caractérisé en ce que** le moyen **(16)** de réception de l'instrument de commande débouche dans une lumière **(6)** aménagée dans une paroi de l'élément femelle **(3)** pour être accessible de l'extérieur de l'implant, la paroi de l'élément femelle **(3)** étant munie intérieurement dans sa partie opposée de celle pourvue de la lumière, de la surface d'ancrage **(10).**

4. Implant de remplacement vertébral selon l'une des revendications 1 à 3, **caractérisé en ce que** chaque élément mâle **(2)** et femelle **(3)** est pourvu d'une paroi **(2ₐ, 3ₐ)** d'extrémité destinée à coopérer avec un plateau vertébral.

5. Implant de remplacement vertébral selon la revendication 4, **caractérisé en ce qu'**au moins une paroi d'extrémité **(2ₐ, 3ₐ)** est équipée d'un système **(30)** d'inclinaison de la paroi d'extrémité dans le plan sagittal.

6. Implant de remplacement vertébral selon la revendication 5, **caractérisé en ce que** le système d'inclinaison **(30)** est du type vis-écrou et comporte une tige filetée **(31)** guidée en rotation et bloquée en translation pour coopérer avec une bague taraudée **(32)** bloquée en rotation et reliée à la paroi d'extrémité guidée en inclinaison.

7. Implant de remplacement vertébral selon la revendication 6, **caractérisé en ce que** la tige filetée **(31)** s'étend diamétralement à l'intérieur d'un élément et comporte un moyen **(48)** de réception d'un instrument de commande accessible de l'extérieur de l'élément.

8. Implant de remplacement selon l'une des revendications 1 à 7, **caractérisé en ce que** chaque élément mâle **(2)** et femelle **(3)** comporte des zones de préhension **(50)** pour un appareil de distraction assurant le coulissement relatif entre les éléments mâle (2) et femelle (3), ces zones de préhension (50) étant réalisées par deux méplats s'étendant de façon diamétralement opposée sur les éléments mâle et femelle.

## Claims

1. Vertebral replacement implant comprising:
• two hollow, longitudinal elements, so-called male (2) and female (3) slidably mounted with respect to one another along a common longitudinal axis (X),
• and means (8) ensuring the locking and free sliding of the elements between each other, the locking means (8) comprising:
- on one of the elements, an anchor surface (10) arranged on a longitudinal part of said element,
- and on the other of the elements:
an anchor shoe (12) guided in translation to take up a first position in which the elements (2, 3) slide freely with respect to each other, and a second position in which the anchor shoe (12) cooperates with the anchor surface (10) to lock the elements (2, 3) together,
a system (15) for converting a rotational movement into a translational movement transmitted to the anchor shoe (12), said conversion system (15) being fitted with means (16) for receiving an instrument commanding rotation in the two opposite directions, so that the anchor shoe (12) is able to take up one of its positions in relation to the direction of the rotational command,
**characterized in that** the conversion system is of screw/nut type (15) and comprises a pin (17) guided in rotation and locked in translation, said pin (17) being provided with a threaded part (25) cooperating with a tapping (26) on the anchor shoe (12) which is guided in translation and locked in rotation.

2. Vertebral replacement implant as in claim 1, **characterized in that** the pin (17) extends diametrically inside the male element (2) and comprises:
• a first end (18) provided with receiving means (16) to receive the command instrument, this first end being guided in rotation and locked in translation in a wall of the male element,
• a second end (27) provided with a threaded part (25) cooperating with the tapping (26) of the anchor shoe which is guided in translation and locked in rotation in the opposite wall of the male element.

3. Vertebral replacement implant as in claim 2, **characterized in that** the means (16) receiving the command instrument lead into a lumen (6) arranged in a wall of the female element (3) so as to be accessible from outside the implant, the wall of the female element (3) being provided on the inside, in its part opposite the part having the lumen, with the anchor surface (10).

4. Vertebral replacement implant as in any of claims 1 to 3, **characterized in that** each male (2) and female (3) element is provided with an end wall (2ₐ, 3ₐ) intended to cooperate with a vertebral plateau.

5. Vertebral replacement implant as in claim 4, **characterized in that** at least one end wall (2ₐ, 3ₐ) is equipped with a system (30) for inclining the end wall in the sagittal plane.

6. Vertebral replacement implant as in claim 5, **characterized in that** the inclining system (30) is of screw/nut type and comprises a threaded rod (31) guided in rotation and locked in translation to cooperate with a tapped ring (32) locked in rotation and connected to the end wall guided in its incline.

7. Vertebral replacement implant as in claim 6, **characterized in that** the threaded rod (31) extends diametrically inside one element and comprises means (48) for receiving a command instrument accessible from outside the element.

8. Replacement implant as in any of claims 1 to 7, **characterized in that** each male (2) and female (3) element comprises grasping zones (50) for a distractor ensuring the relative sliding between the male (2) and female (3) elements, these grasping zones (50) being formed of two flats extending in diametrical opposite manner on the male and female elements.

## Patentansprüche

1. Wirbelkörperersatz-Implantat mit:
• zwei hohlen Längselementen, einem sogenannten einzusteckenden (2) und einem sogenannten aufnehmenden (3), die entlang einer gemeinsamen Längsachse (X) verschieblich zueinander angebracht sind, sowie
• Mittel (8), welche die Blockierung und das freie Verschieben der Elemente untereinander sicherstellen, wobei die Blockierungsmittel (8) folgendes aufweisen:
- an einem der Elemente eine Verankerungsfläche (10), die an einem Längsteil des Elements ausgebildet ist, und
- an dem anderen der Elemente:
• einen Verankerungsschuh (12), der verschiebegeführt ist, um eine erste Position einzunehmen, in der die Elemente (2, 3) untereinander frei verschieblich sind, sowie eine zweite Position, in welcher der Verankerungsschuh (12) mit der Verankerungsfläche (10) zusammenwirkt, um die Elemente (2, 3) untereinander zu blockieren,
• ein System (15) zur Umwandlung einer Drehbewegung in eine Verschiebebewegung, die auf den Verankerungsschuh (12) übertragen wird, wobei das Bewegungsumwandlungssystem (15) mit einem Mittel (16) zur Aufnahme eines Instruments zur Drehbetätigung in die beiden entgegengesetzten Richtungen versehen ist, so daß der Verankerungsschuh (12) in Abhängigkeit der Richtung der Drehbetätigung eine seiner Positionen einnimmt,
**dadurch gekennzeichnet, daß** das Umwandlungssystem von der Art Schraube-Mutter (15) ist und eine drehgeführte und gegen Verschiebung festgelegte Achse (17) aufweist, wobei diese Achse (17) mit einem Gewindeteil (25) versehen ist, der mit einem Innengewinde (26) des verschiebegeführten und gegen Drehung festgelegten Verankerungsschuhs (12) zusammenwirkt.

2. Wirbelkörperersatz-Implantat nach Anspruch 1, **dadurch gekennzeichnet, daß** die Achse (17) diametral innerhalb des Einsteckelements (2) verläuft und folgendes umfaßt:
• ein erstes Ende (18), das mit dem Mittel (16) zur Aufnahme des Betätigungsinstruments versehen ist, wobei dieses erste Ende in einer Wand des Einsteckelements drehgeführt und gegen Verschiebung festgelegt ist,
• ein zweites Ende (27), das mit dem Gewindeteil (25) versehen ist, der mit dem Innengewinde (26) des Verankerungsschuhs zusammenwirkt, welcher in der gegenüberliegenden Wand des Einsteckelements verschiebegeführt und gegen Drehung festgelegt ist.

3. Wirbelkörperersatz-Implantat nach Anspruch 2, **dadurch gekennzeichnet, daß** das Mittel (16) zur Aufnahme des Betätigungsinstruments in eine Öffnung (6) mündet, die in einer Wand des Aufnahmeelements (3) ausgebildet ist, um von der Außenseite des Implantats aus zugänglich zu sein, wobei die Wand des Aufnahmeelements (3) innen, in ihrem Teil, der dem mit der Öffnung versehenen gegenüberliegt, mit der Verankerungsfläche (10) ausgestattet ist.

4. Wirbelkörperersatz-Implantat nach einem der Ansprüche 1 bis 3, **dadurch gekennzeichnet, daß** jedes Einsteckelement (2) und Aufnahmeelement (3) mit einer endseitigen Wand (2ₐ, 3ₐ) versehen ist, die dazu bestimmt ist, mit einer Wirbelplatte zusammenzuwirken.

5. Wirbelkörperersatz-Implantat nach Anspruch 4, **dadurch gekennzeichnet, daß** wenigstens eine endseitige Wand (2ₐ, 3ₐ) mit einem System (30) zur Neigung der endseitigen Wand in der Sagittalebene ausgestattet ist.

6. Wirbelkörperersatz-Implantat nach Anspruch 5, **dadurch gekennzeichnet, daß** das Neigungssystem (30) von der Art Schraube-Mutter ist und einen drehgeführten und gegen Verschiebung festgelegten Gewindestift (31) umfaßt, um mit einem mit innengewinde versehenen Ring (32) zusammenzuwirken, der gegen Drehung festgelegt und mit der neigungsgeführten endseitigen Wand verbunden ist.

7. Wirbelkörperersatz-Implantat nach Anspruch 6, **dadurch gekennzeichnet, daß** der Gewindestift (31) diametral innerhalb eines Elements verläuft und ein Mittel (48) zur Aufnahme eines von der Außenseite des Elements zugänglichen Betätigungsinstruments umfaßt.

8. Ersatz-Implantat nach einem der Ansprüche 1 bis 7, **dadurch gekennzeichnet, daß** jedes Einsteckelement (2) und Aufnahmeelement (3) Greifbereiche (50) für ein Trenngerät aufweist, welches das Relatiwerschieben zwischen dem Einsteckelement (2) und dem Aufnahmeelement (3) sicherstellt, wobei diese Greifbereiche (50) durch zwei Abflachungen realisiert sind, die sich diametral gegenüberliegend an dem Einsteck- und dem Aufnahmeelement erstrecken.
